# EUROPÄISCHE PATENTSCHRIFT

(11) **EP 2 822 608 B1**
(45) Veröffentlichungstag und Bekanntmachung des Hinweises auf die Patenterteilung: **10.01.2018**
(21) Anmeldenummer: 13705768.3
(22) Anmeldetag: 22.02.2013
(51) Int. Cl.: A61L 2/26, A61B 50/30

(54) **CHIRURGISCHE STERILISIERBEHÄLTERWANNE MIT KONDENSATABLEITUNG**
SURGICAL STERILISATION CONTAINER WITH CONDENSATE OUTLET
CUVE POUR RÉCIPIENT DE STÉRILISATION À USAGE CHIRURGICAL AVEC SORTIE DE CONDENSE

(30) Priorität: 05.03.2012 DE 102012101833
(43) Veröffentlichungstag der Anmeldung: 14.01.2015
(73) Patentinhaber: Aesculap AG, 78532 Tuttlingen (DE)
(72) Erfinder: GRAY-DREIZLER, John, 78628 Rottweil (DE); GLEICHAUF, Wilhelm, 78532 Tuttlingen-Möhringen (DE); JAKAB, Mariana, 78532 Tuttlingen (DE); THOMAS, Stefan, 78532 Tuttlingen (DE); SCHUSTER, Stefan, 78048 Villingen-Schwenningen (DE)
(74) Vertreter: Hoeger, Stellrecht & Partner Patentanwälte mbB
(86) Internationale Anmeldenummer: PCT/EP2013/053533
(87) Internationale Veröffentlichungsnummer: WO 2013/131761

(56) Entgegenhaltungen:
- EP-A1- 1 016 369
- WO-A1-2012/038314
- WO-A2-99/27969
- DE-A1-102004 028 040
- FR-A1- 2 542 200
- US-A- 3 437 423
- US-A- 4 671 943
- US-A- 4 900 519
- US-A- 5 441 707
- US-A- 5 971 152
- US-B1- 6 821 286

## Beschreibung

Die Erfindung betrifft eine chirurgische Sterilisierbehälterwanne, auf die ein Sterilisierbehälterdeckel lösbar aufsetzbar ist zum Ausbilden eines chirurgischen Sterilisierbehälters, in dem chirurgische Instrumente zum Sterilisieren aufnehmbar sind, wobei die Sterilisierbehälterwanne einen Boden sowie eine vom Boden abstehende Behälterwand umfasst, wobei die Sterilisierbehälterwanne eine Aufstellebene definiert und wobei der Boden einen Fluidsammelbereich zum Sammeln von Fluid umfasst oder ausbildet.

Außerdem betrifft die Erfindung einen chirurgischen Sterilisierbehälter, umfassend eine chirurgische Sterilisierbehälterwanne und einen auf die Sterilisierbehälterwanne lösbar aufsetzbaren Sterilisierbehälterdeckel.

Es sind Sterilisierbehälter mit Sterilisierbehälterwannen der eingangs genannten Art bekannt, in deren Bodenmitte ein muldenförmiger Fluidsammelbereich angeordnet ist. Am Fluidsammelbereich ist eine Durchgangsöffnung im Boden gebildet, an der ein druck- oder temperaturgesteuertes Auslassventil angeordnet ist, mit dem die Durchgangsöffnung verschlossen und freigegeben werden kann. Fluid wie insbesondere sich während des Sterilisiervorgangs bildendes Kondensat kann bei geöffnetem Auslassventil durch den Boden aus dem Sterilisierbehälter abgelassen werden. Dies birgt allerdings den großen Nachteil in sich, dass abgelassenes Kondensat schwallartig aus dem Sterilisierbehälter austritt. Im Falle übereinander gestapelter Sterilisierbehälter kann das Kondensat vom höher gelegenen Sterilisierbehälter über den Deckel des darunter gelegenen Sterilisierbehälters fließen. Dies führt zu einer unerwünschten Abkühlung des tiefer gelegenen Sterilisierbehälters und unerwünschter Kondensatnachbildung in dessen Behälterinnenraum. Das Kondensat muss zusätzlich abgelassen oder während der Trocknungsphase zusätzlich verdampft werden.

Darüber hinaus erweist sich das Vorsehen einer Durchgangsöffnung im Boden als nachteilig. Bei einer Fehlfunktion oder Beschädigung des Auslassventils ist der Sterilisierbehälter unzureichend abgedichtet. Dies kann zu einem unzureichenden Sterilisierergebnis führen und das Eintreten von Keimen in den Behälterinnenraum begünstigen. Eine unerwünschtes Öffnen, eine Beschädigung oder eine Fehlfunktion des Auslassventils kann beispielsweise durch Unebenheiten einer Aufstellfläche oder durch Gegenstände auf einer Aufstellfläche für den Sterilisierbehälter hervorgerufen werden, die von unten auf das Auslassventil einwirken. Als nachteilig erweist sich dabei auch, dass aufgrund der Anordnung der Öffnung im Boden eine Beschädigung des Auslassventils für üblicherweise mit dem Sterilisierbehälter hantierendes Krankenhauspersonal schwer erkennbar ist.

"Aufstellfläche" ist eine von der Sterilisierbehälterwanne definierte Kontaktebene, in der die Sterilisierbehälterwanne eine Aufstellfläche kontaktiert, auf welcher die Sterilisierbehälterwanne aufgestellt wird. Die üblicherweise horizontal ausgerichtete Aufstellfläche führt bei bestimmungsgemäßem Gebrauch der Sterilisierbehälterwanne in einer Betriebsstellung zu einer horizontalen Ausrichtung der Aufstell- oder Kontaktebene.

Unterschiedliche Ausführungsformen chirurgischer Sterilisierbehälter sind in den Druckschriften EP 1 016 369 A1, US 5,971,152, WO 99/27969 A2, US 4,671,943, US 3,437,423, US 4,900,519, WO 2012/038314 A1, DE 10 2004 028 040 A1, US 6,821,286 B1, US 5,441,707 und FR 2 542 200 beschrieben.

Aufgabe der vorliegenden Erfindung ist es, eine chirurgische Sterilisierbehälterwanne und einen chirurgischen Sterilisierbehälter der eingangs genannten Art bereitzustellen, mit der bzw. dem zu entfernendes Fluid dem Fluidsammelbereich wirksam zugeführt werden kann.

Diese Aufgabe wird erfindungsgemäß durch eine Sterilisierbehälterwanne mit den Merkmalen von Anspruch 1 gelöst.

Bei der erfindungsgemäßen Sterilisierbehälterwanne ist eine Ableitfläche vorgesehen, die relativ zur Aufstellebene geneigt ist. Darunter ist insbesondere zu verstehen, dass die Ableitfläche nicht in der Aufstellebene liegt oder parallel zu dieser ausgerichtet ist. Die Ableitfläche erlaubt es, Fluid in den Fluidsammelbereich zu leiten, und zwar in Richtung der Behälterwand der Sterilisierbehälterwanne. Dies ermöglicht es, Fluid wie insbesondere während des Sterilisiervorgangs gebildetes Kondensat speziell weg von der Bodenmitte in Richtung der Behälterwand und in den Fluidsammelbereich zu leiten. Auf diese Weise verringert sich der Abstand des mit der Ableitfläche abgeleiteten Fluids zur Behälterwand. Dies gibt zum Beispiel die Möglichkeit, Restwärme der Behälterwand während der Trocknungsphase nach dem Sterilisiervorgang wirkungsvoller zu nutzen als bei einer herkömmlichen Sterilisierbehälterwanne, da die Verdampfung von Fluid durch die Nähe zur Behälterwand begünstigt ist. Das Ableiten des Fluids durch die Fluidableitfläche in Richtung der Behälterwand und dessen Sammeln im Fluidsammelbereich erlaubt es auch, das Fluid auf andersartige Weise aus der Sterilisierbehälterwanne zu entfernen als bei den vorstehend beschriebenen Sterilisierbehältern. Hierzu kann beispielsweise eine in der Behälterwand gebildete Durchgangsöffnung vorgesehen sein, an der ein Auslassventil angeordnet ist zum Freigeben und Schließen der Durchgangsöffnung. In Richtung der Behälterwand abgeleitetes Fluid kann aus der Sterilisierbehälterwanne in diesem Fall bei geöffnetem Auslassventil seitlich durch die Behälterwand hindurch austreten. Die Nachteile des vorstehend beschriebenen Sterilisierbehälters, bei dem Fluid nach unten abgelassen wird, lassen sich auf diese Weise weitgehend vermeiden.

Speziell bei der zuletzt erläuterten Ausführungsform der Sterilisierbehälterwanne, bei der in der Behälterwand eine Durchgangsöffnung gebildet ist, ist es günstig, wenn der Boden am Fluidsammelbereich frei von Durchgangsöffnungen ist. Dies erlaubt es, durch den Boden am Fluidsammelbereich eine Sterilbarriere auszubilden und eine Leckage der Sterilisierbehälterwanne zu vermeiden.

Die Größe der Fluidableitfläche beträgt mindestens 20 % der Bodenfläche.

Günstig ist es, dass die Sterilisierbehälterwanne einen rechteckigen oder im Wesentlichen rechteckigen Querschnitt aufweist mit vier die Behälterwand bildenden Seitenwänden, und dass die Fluidableitfläche in Richtung einer der Seitenwände geneigt ist zum Ableiten von Fluid in Richtung der Seitenwand. Als "in Richtung einer der Seitenwände geneigt" kann insbesondere verstanden werden, dass der Abstand der Fluidableitfläche von der Aufstellebene an einem der Seitenwand zugewandten Abschnitt der Fluidableitfläche kleiner ist als an einem der Seitenwand abgewandten Abschnitt der Fluidableitfläche. Bei dieser Ausführungsform ist die Möglichkeit gegeben, Fluid in Richtung einer der Seitenwände abzuleiten. Insbesondere kann vorgesehen sein, dass in dieser Seitenwand eine mittels eines Auslassventils verschließbare Durchgangsöffnung gebildet ist, durch die hindurch im Fluidsammelbereich gesammeltes Fluid aus der Sterilisierbehälterwanne austreten kann.

Der Fluidsammelbereich ist derjenigen Seitenwand benachbart, in Richtung derer die Fluidableitfläche zum Ableiten von Fluid geneigt ist. Dadurch kann sichergestellt werden, dass Fluid möglichst nah bis an die Seitenwand von der Ableitfläche geleitet werden kann. Unter "benachbart" kann vorliegend insbesondere verstanden werden, dass der Fluidsammelbereich längs der Seitenwand verläuft und insbesondere in direkter Fluidverbindung mit der Behälterwand steht.

Vorzugsweise ist der Boden frei von Durchgangsöffnungen, auch außerhalb des Fluidsammelbereichs. Dadurch kann der Boden insgesamt eine Sterilbarriere bilden, und eine Leckage der Sterilisierbehälterwanne wird vermieden.

Bei einer Umsetzung einer vorteilhaften Sterilisierbehälterwanne erweist es sich als günstig, wenn die Größe der Fluidableitfläche mindestens 30 % der Bodenfläche beträgt.

Beispielsweise erweist es sich bei einer bevorzugten Ausführungsform der Sterilisierbehälterwanne als günstig, wenn die Größe der Fluidableitfläche ungefähr ein Drittel der Bodenfläche beträgt.

Bei einer andersartigen vorteilhaften Ausführungsform hat es sich als günstig erwiesen, wenn die Größe der Fluidableitfläche ungefähr 40 % bis ungefähr 50 % der Bodenfläche beträgt.

Günstig erweist es sich bei einer Umsetzung der Sterilisierbehälterwanne in der Praxis, wenn die Neigung der Fluidableitfläche relativ zur Aufstellebene kleiner ist als 10°, um günstigerweise eine geringe Bauhöhe des Bodens zu erzielen. Als bevorzugt hat sich eine Neigung der Fluidableitfläche relativ zur Aufstellebene kleiner als 5° erwiesen, noch bevorzugter kleiner als 2°.

Bei einer vorteilhaften Ausführungsform der erfindungsgemäßen Sterilisierbehälterwanne hat es sich als günstig erwiesen, wenn die Neigung der Fluidableitfläche relativ zur Aufstellebene ungefähr 1,5° beträgt.

Vorzugsweise ist der Fluidsammelbereich durch eine Vertiefung des Bodens gebildet. Dies gibt die Möglichkeit einer konstruktiv einfachen Ausgestaltung der Sterilisierbehälterwanne.

Von Vorteil ist es, wenn die Sterilisierbehälterwanne eine den Boden bildende Bodenwand umfasst und wenn die Bodenwand die Fluidableitfläche und/oder den Fluidsammelbereich ausbildet. Dies erlaubt es, eine konstruktiv einfache Ausgestaltung der Sterilisierbehälterwanne zu erzielen. Insbesondere kann dadurch ein auf einer Bodenwand der Sterilisierbehälterwanne positionierbarer Bodeneinsatz eingespart werden.

Die Bodenwand ist bevorzugt zumindest am Fluidsammelbereich und günstigerweise insgesamt frei von Öffnungen, um eine Sterilbarriere auszubilden und eine Leckage der Sterilisierbehälterwanne zu vermeiden.

Von Vorteil ist es, wenn der Fluidsammelbereich außermittig am Boden angeordnet ist. Dies kann insbesondere derart aufgefasst werden, dass der Fluidsammelbereich einen Abstand zu einer Bodenmitte aufweist. Dies erlaubt es, mittels der Fluidableitfläche abgeleitetes Fluid möglichst nah an die Behälterwand abzuleiten.

Vorzugsweise steht der Fluidsammelbereich in direkter Fluidverbindung mit der Behälterwand. Dies gibt die Möglichkeit, Fluid bis direkt an die Behälterwand abzuleiten. Beispielsweise bildet die Behälterwand abschnittsweise eine Wand des Fluidsammelbereiches.

Als vorteilhaft erweist es sich, wenn der Fluidsammelbereich längs eines Abschnittes der Behälterwand verläuft, beispielsweise zur Ausgestaltung der vorstehend erwähnten bevorzugten Ausführungsform. Darunter wird insbesondere verstanden, dass der Fluidsammelbereich längs eines Abschnittes der Behälterwand angeordnet ist und/oder dass der Fluidsammelbereich einem Abschnitt der Behälterwand benachbart ist. Insbesondere kann darunter verstanden werden, dass der Fluidsammelbereich in direkter Fluidverbindung mit einem Abschnitt der Behälterwand steht.

Es ist vorteilhafterweise vorgesehen, dass die Fluidableitfläche in Richtung nur einer der Seitenwände der Behälterwand geneigt ist, so dass Fluid nach Möglichkeit weitgehend nur in Richtung einer der Seitenwände abgeleitet wird.

Als vorteilhaft erweist es sich, wenn die Behälterwand zwei Längsseitenwände und zwei Querseitenwände umfasst und wenn die Fluidableitfläche in Richtung einer Querseitenwand geneigt ist zum Ableiten von Fluid in Richtung der Querseitenwand. Bei dieser Ausführungsform ist die Möglichkeit gegeben, Fluid in Richtung einer Quer- und damit einer Schmalseite der Sterilisierbehälterwanne abzuleiten. Dies gibt die Möglichkeit einer kompakteren Ausgestaltung des Fluidsammelbereiches, insbesondere bei dessen Anordnung längs der oder benachbart zur Querseitenwand. In der Querseitenwand kann eine mittels eines Auslassventils verschließbare Durchgangsöffnung gebildet sein, über die Fluid aus der Sterilisierbehälterwanne austreten kann.

Vorzugsweise erstreckt sich die Fluidableitfläche über die Mitte des Bodens hinweg, so dass Fluid auch von der Bodenmitte in Richtung der Behälterwand abgeleitet werden kann. Beispielsweise kann bei einer rechteckförmigen Sterilisierbehälterwanne vorgesehen sein, dass sich die Fluidableitfläche entlang einer Längsrichtung über ungefähr 60 % bis ungefähr 70 % des Bodens erstreckt und in einer Querrichtung über ungefähr 45 % bis ungefähr 55 % der Bodenfläche, wobei sich die Fluidableitfläche über deren Mitte hinweg erstreckt.

Für eine konstruktiv einfache Ausgestaltung erweist es sich als günstig, wenn die Fluidableitfläche planar ausgebildet ist. Darüber hinaus kann Fluid über die planare Fluidableitfläche zuverlässig in den Fluidsammelbereich abgeleitet werden.

Wie bereits erwähnt, ist bei einer bevorzugten Ausführungsform der erfindungsgemäßen Sterilisierbehälterwanne vorgesehen, dass in der Behälterwand eine Durchgangsöffnung gebildet ist und dass die Sterilisierbehälterwanne ein Auslassventil umfasst oder ein solches an der Sterilisierbehälterwanne festgelegt ist, mit dem die Durchgangsöffnung freigebbar und verschließbar ist. Durch die Durchgangsöffnung hindurch kann dem Fluidsammelbereich zugeleitetes Fluid aus der Sterilisierbehälterwanne austreten. Zu diesem Zweck ist der Fluidsammelbereich bevorzugt einem die Durchgangsöffnung aufweisenden Abschnitt der Behälterwand benachbart oder verläuft längs dieses Abschnittes.

Als vorteilhaft erweist es sich, wenn die Sterilisierbehälterwanne eine Fluidhebeeinrichtung umfasst oder eine solche an der Sterilisierbehälterwanne festgelegt ist zum Bereitstellen einer Fluidverbindung vom Fluidsammelbereich zum Auslassventil und zum Anheben von Fluid vom Fluidsammelbereich zum Auslassventil, wobei das Auslassventil günstigerweise im Abstand zum Boden an der Behälterwand angeordnet ist. Die Fluidhebeeinrichtung gibt die Möglichkeit, gesammeltes Fluid zum Auslassventil anzuheben, so dass dieses aus der Sterilisierbehälterwanne austreten kann. Beispielsweise umfasst die Fluidhebeeinrichtung einen Fluidkanal, der in den Fluidsammelbereich eingreift mit einer Fluideintrittsöffnung. Der Fluidkanal kann eine Fluidaustrittsöffnung umfassen, die beispielsweise an einer Aufstromseite des Auslassventils angeordnet oder auf diese gerichtet ist. Bei Öffnen des Auslassventils kann sich eine Strömung durch die Durchgangsöffnung hindurch ausbilden, unter deren Wirkung Fluid durch den Fluidkanal hindurch zum Auslassventil angehoben wird und aus der Sterilisierbehälterwanne austreten kann.

Wie bereits erwähnt, ist die Fluidableitfläche bevorzugt in Richtung nur einer Seitenwand der Sterilisierbehälterwanne geneigt, in Richtung derer Fluid abgeleitet werden kann. Dies kann beispielsweise durch eine asymmetrische Ausgestaltung des Bodens erzielt werden.

Insgesamt ist es von Vorteil, wenn die Sterilisierbehälterwanne eine senkrecht zur Aufstellebene ausgerichtete Wannenmittenebene definiert, bezüglich derer der Boden asymmetrisch ausgebildet ist. Beispielsweise kann sich die Fluidableitfläche über eine Mitte des Bodens erstrecken und dadurch diese Asymmetrieebene schneiden, um Fluid dem im Abstand zur Asymmetrieebene angeordneten Fluidsammelbereich zuzuführen.

Insbesondere ist der Fluidsammelbereich asymmetrisch bezüglich der Asymmetrieebene.

Alternativ oder ergänzend kann vorgesehen sein, dass die Sterilisierbehälterwanne eine senkrecht zur Aufstellebene ausgerichtete Wannenmittenebene definiert, bezüglich derer der Boden symmetrisch ausgebildet ist. Dies ermöglicht eine konstruktive Vereinfachung der Sterilisierbehälterwanne. Die Symmetrieebene des Bodens kann insbesondere senkrecht zu der vorstehend erwähnten Asymmetrieebene des Bodens ausgerichtet sein.

Als vorteilhaft erweist es sich, wenn der Boden Vertiefungen umfasst oder ausbildet, im Bereich derer die Sterilisierbehälterwanne außenseitig Aufstellelemente aufweist, die die Aufstellebene definieren. Durch die Ausbildung der Vertiefungen im Boden können sich außenseitig an der Sterilisierbehälterwanne insbesondere Erhebungen ausbilden, welche die Aufstellebene definieren und als Aufstellelemente wirken können, über welche die Sterilisierbehälterwanne eine Aufstellfläche kontaktieren kann. Gesonderte Aufstellelemente, die von der Außenseite mit dem Boden verbunden werden, können dadurch eingespart werden. Bei dieser Ausführungsform erweist es sich als vorteilhaft, wenn der Boden durch eine Bodenwand gebildet ist.

Günstig ist es, wenn die Vertiefungen, die einem Abschnitt der Behälterwand benachbart sind, in Richtung dessen die Fluidableitfläche geneigt ist, einen Teil des Fluidsammelbereichs ausbilden. Dies erlaubt eine konstruktive Vereinfachung der Sterilisierbehälterwanne. Die Vertiefungen können Fluid aufnehmen, das von der Fluidableitfläche in Richtung der Behälterwand abgeleitet wird und dadurch einen Teil des Fluidsammelbereichs bilden.

Es kann vorgesehen sein, dass der vorstehend erwähnte Fluidkanal der Fluidhebeeinrichtung in eine der Vertiefungen eingreift, so dass gesammeltes Fluid in den Fluidkanal eintreten kann.

Vorteilhafterweise kann vorgesehen sein, dass die Sterilisierbehälterwanne einen rechteckigen oder im Wesentlichen rechteckigen Querschnitt aufweist mit vier die Behälterwand bildenden Seitenwänden und Vertiefungen an Eckbereichen der Sterilisierbehälterwanne, wobei die Fluidableitfläche in Richtung einer der Seitenwände geneigt ist, und dass zumindest eine der der Seitenwand benachbarten Vertiefungen einen Teil des Fluidsammelbereichs ausbildet. Mindestens eine, vorzugsweise zwei der vier Vertiefungen an den Eckbereichen der Sterilisierbehälterwanne kann/können einen Teil des Fluidsammelbereichs ausbilden, der zum Beispiel längs der Seitenwand verläuft, in Richtung auf die die Fluidableitfläche geneigt ist.

Vorzugsweise weist der Boden am Fluidsammelbereich einen parallel zur Aufstellebene ausgerichteten Bodenabschnitt auf, der im Abstand zur Behälterwand mit der Fluidableitfläche verbunden ist. Die Fluidableitfläche reicht bei dieser bevorzugten Ausführungsform nicht direkt bis zur Behälterwand, sondern bis zu einem parallel zur Aufstellebene ausgerichteten Bodenabschnitt. Dadurch braucht sich die geneigte Fluidableitfläche nicht bis zur Bodenwand zu erstrecken. Dies gibt die Möglichkeit, eine geringe Bauhöhe des Bodens zu erzielen.

Der parallel zur Aufstellebene ausgerichtete Bodenabschnitt kann in eine oder mehrere der vorstehend erwähnten Vertiefungen des Bodens übergehen, die einen Teil des Fluidsammelbereichs ausbildet/ausbilden.

Als günstig erweist es sich, wenn sich der Bodenabschnitt von der Fluidableitfläche bis zur oder im Wesentlichen bis zur Behälterwand erstreckt. Beispielsweise kann der Bodenabschnitt auf der der Fluidableitfläche abgewandten Seite mit der Behälterwand verbunden sein.

In Abwandlung der zuletzt beschriebenen vorteilhaften Ausführungsformen der erfindungsgemäßen Sterilisierbehälterwanne kann vorgesehen sein, dass sich die Fluidableitfläche direkt bis zur Behälterwand erstreckt.

Günstig ist es, wenn der Boden Tragelemente umfasst oder ausbildet zum Tragen einer Aufnahme für chirurgische Instrumente und wenn die Tragelemente eine parallel zur Aufstellebene ausgerichtete Tragebene definieren. Auf den Tragelementen kann eine Aufnahme für Instrumente, insbesondere einen chirurgischen Siebkorb, positioniert werden. Die Tragebene ermöglicht eine zuverlässige Positionierung und damit eine zuverlässige Abstützung der Aufnahme.

Vorzugsweise weist die Tragebene einen gleich großen oder einen größeren Abstand zur Aufstellebene auf als die Fluidableitfläche. Darunter kann insbesondere verstanden werden, dass die Fluidableitfläche nicht über die Tragebene hinaus in einen von der Sterilisierbehälterwanne definierten Aufnahmeraum ragt. Dies erlaubt zum Einen zuverlässiges Positionieren der Aufnahme für chirurgische Instrumente auf den Tragelementen. Zum Anderen kann Fluid unterhalb der Aufnahme abgeleitet werden.

Bei einer konstruktiv einfachen Ausgestaltung ist es günstig, wenn die Tragelemente planar ausgestaltet sind. Beispielsweise können die Tragelemente parallel zur Aufstellebene verlaufende Abschnitte des Bodens und insbesondere einer Bodenwand der Sterilisierbehälterwanne sein.

Günstig ist es, wenn der Boden zwei Tragelemente aufweist, die längs zweier im Abstand zueinander angeordneter Seitenwände der Sterilisierbehälterwanne verlaufen, und wenn die Fluidableitfläche zwischen den Tragelementen angeordnet ist. Dadurch kann die Aufnahme außenseitig auf den vorzugsweise planaren Tragelementen abgestellt werden. Fluid, etwa Kondensat, kann unterhalb der Aufnahme von der Fluidableitfläche abgeleitet werden. Die Tragelemente sind bevorzugt Bodenabschnitte, die entlang von Längsseiten der Behälterwand in einer Längsrichtung der Sterilisierbehälterwanne verlaufen, wobei die Fluidableitfläche in deren Querrichtung zwischen den Tragelementen angeordnet ist. Günstigerweise ist die Fluidableitfläche in Richtung einer Querseitenwand der Behälterwand geneigt.

Wie eingangs erwähnt, betrifft die Erfindung auch einen chirurgischen Sterilisierbehälter. Bei einem chirurgischen Sterilisierbehälter der eingangs genannten Art, der eine Sterilisierbehälterwanne und einen auf die Sterilisierbehälterwanne lösbar aufsetzbaren Sterilisierbehälterdeckel umfasst, wird die eingangs genannte Aufgabe erfindungsgemäß dadurch gelöst, dass der Sterilisierbehälter eine Sterilisierbehälterwanne der vorstehend beschriebenen Art aufweist.

Die bereits im Zusammenhang mit der erfindungsgemäßen Sterilisierbehälterwanne erwähnten Vorteile können mit dem erfindungsgemäßen Sterilisierbehälter erzielt werden, so dass diesbezüglich auf vorstehende Erläuterungen verwiesen werden kann.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine perspektivische Darstellung eines erfindungsgemäßen Sterilisierbehälters im geöffneten Zustand, umfassend eine erfindungsgemäße Sterilisierbehälterwanne und eine Fluid-extraktionsvorrichtung;
- Figur 2:: eine perspektivische Darstellung der Fluidextraktionsvorrich-tung des Sterilisierbehälters aus Figur 1;
- Figur 3:: eine Schnittansicht längs der Linie 3-3 in Figur 1;
- Figur 4:: eine Draufsicht auf die Sterilisierbehälterwanne des Sterilisier-behälters aus Figur 1;
- Figur 5:: eine Schnittansicht längs der Linie 5-5 in Figur 4;

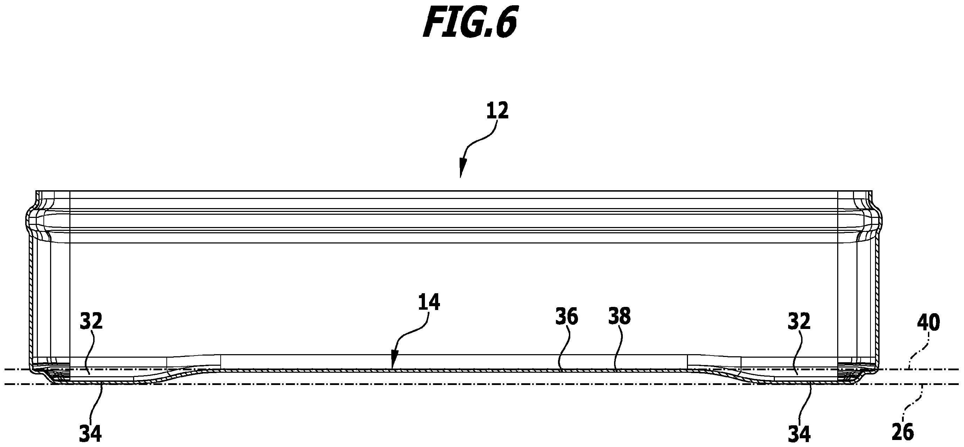

- Figur 6:: eine Schnittansicht längs der Linie 6-6 in Figur 4 und
- Figur 7:: eine perspektivische Darstellung der Sterilisierbehälterwanne des Sterilisierbehälters aus Figur 1.

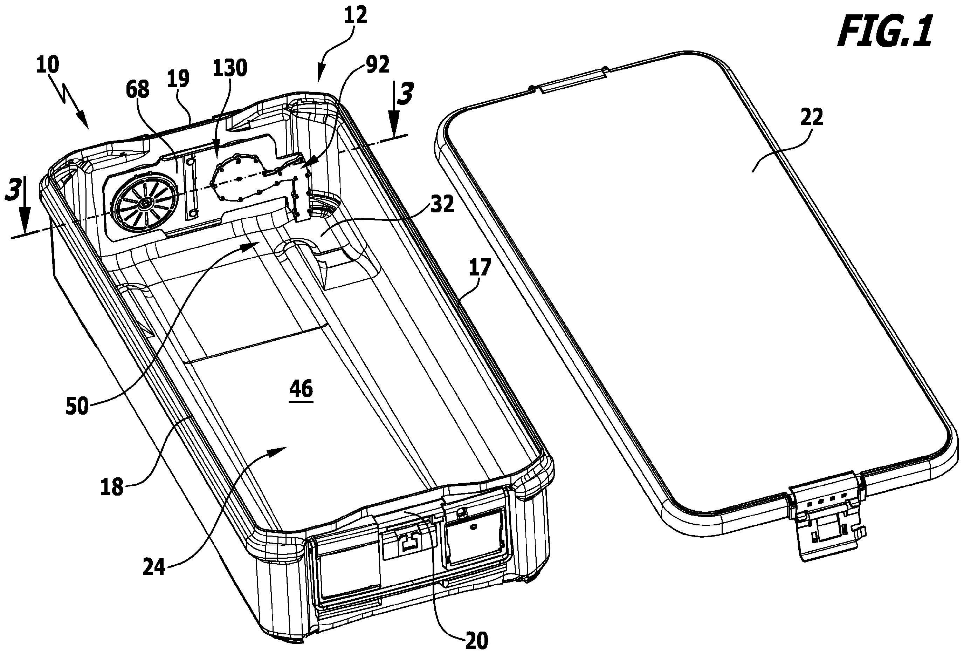

Figur 1 zeigt in perspektivischer Darstellung eine insgesamt mit dem Bezugszeichen 10 belegte bevorzugte Ausführungsform eines erfindungsgemäßen Sterilisierbehälters. Der Sterilisierbehälter 10 dient zur Aufnahme von chirurgischen Instrumenten während des Sterilisiervorgangs. Die in der Zeichnung nicht dargestellten Instrumente sind üblicherweise in einer in der Zeichnung ebenfalls nicht dargestellten Aufnahme angeordnet, beispielsweise in einem chirurgischen Siebkorb, der im Sterilisierbehälter 10 aufgenommen ist.

Der Sterilisierbehälter 10 umfasst eine bevorzugte Ausführungsform einer erfindungsgemäßen Sterilisierbehälterwanne 12 von im Wesentlichen rechteckförmiger Gestalt, die einen Boden 14 umfasst sowie eine vom Boden 14 abstehende Außenwand 16. Der Boden 14 wird gebildet durch eine Bodenwand 15. Die Außenwand 16 umfasst vier Seitenwände, nämlich zwei Längsseitenwände 17 und 18, die endseitig mittels zweier Querseitenwände 19 bzw. 20 miteinander verbunden sind. Die Außenwand 16 ist eine Behälterwand des Sterilisierbehälters 10. Die Längsseitenwände 17 und 18 definieren eine Längsrichtung des Sterilisierbehälters 10, die Querseitenwände 19 und 20 definieren dessen Querrichtung.

Der Sterilisierbehälter 10 weist einen Sterilisierbehälterdeckel 22 auf, der dichtend auf die Sterilisierbehälterwanne 12 aufgesetzt werden kann, um diese abzudecken und einen vom Sterilisierbehälter 10 definierten Behälterinnenraum 24 zu verschließen. Mittels an sich bekannter Verschlusselemente kann der Sterilisierbehälterdeckel 22 mit der Sterilisierbehälterwanne 12 lösbar verbunden werden.

Die Sterilisierbehälterwanne 12 definiert eine Aufstellebene 26 des Sterilisierbehälters 10, bei der es sich um eine Kontaktebene handelt, in der die Sterilisierbehälterwanne 12 eine Aufstellfläche kontaktiert, auf welcher sie aufgestellt wird. Bei üblicherweise horizontal ausgerichteter Aufstellfläche ist die Aufstellebene 26 horizontal ausgerichtet.

Positions- und Orientierungsangaben wie beispielsweise "oben", "unten" oder dergleichen beziehen sich vorliegend auf eine Gebrauchsstellung des Sterilisierbehälters 10 auf einer horizontalen Aufstellfläche und damit horizontal ausgerichteter Aufstellebene 26 in einer Betriebsstellung des Sterilisierbehälters 10.

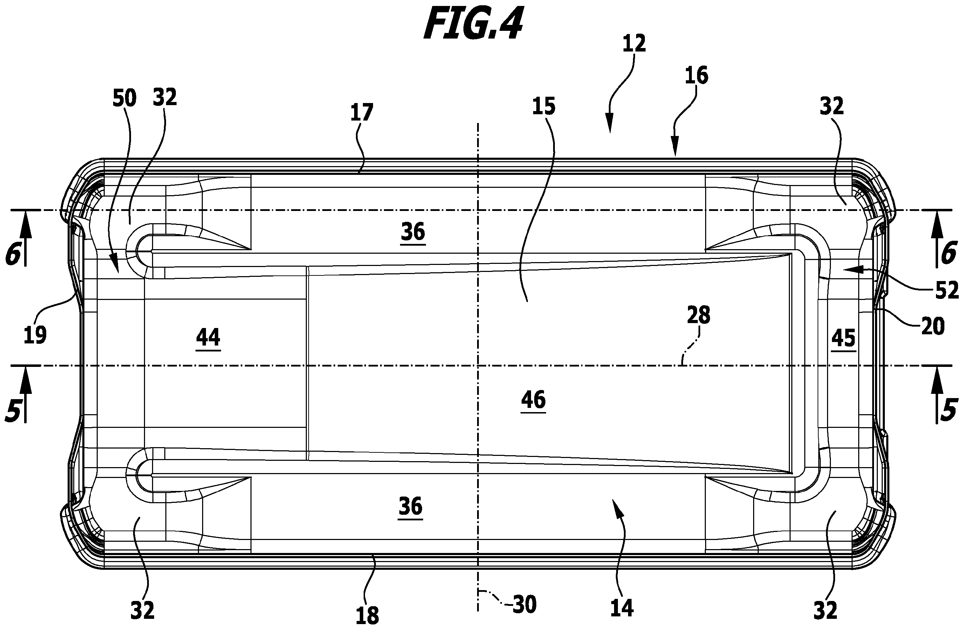

Die Sterilisierbehälterwanne 12, speziell der Boden 14, ist insgesamt symmetrisch ausgebildet bezüglich einer Symmetrieebene 28, die senkrecht zur Aufstellebene 26 ausgerichtet ist und eine Wannenmittenebene ist. Die Symmetrieebene 28 verläuft mittig zwischen den Längsseitenwänden 17 und 18, in Figur 4 senkrecht zur Zeichenebene und längs der Linie 5-5.

Weiter ist die Sterilisierbehälterwanne 12, speziell der Boden 14, asymmetrisch ausgebildet bezüglich einer Asymmetrieebene 30, die senkrecht zur Aufstellebene 26 und senkrecht zur Symmetrieebene 28 ausgerichtet ist und bei der es sich um eine Wannenmittenebene handelt, die mittig zwischen den Querseitenwänden 19 und 20 verläuft.

An Eckbereichen der Sterilisierbehälterwanne 12, an denen die Längsseitenwände 17, 18 und die Querseitenwände 19, 20 aufeinander stoßen, sind im Boden 14 Vertiefungen 32 vorhanden. Die Vertiefungen 32 werden, wie der Boden 14 im Übrigen, beim Formen der Sterilisierbehälterwanne 12 mittels eines Umformverfahrens gebildet, beispielsweise durch Tiefziehen. Durch Ausbilden der Vertiefungen 32 weist der Boden 14 außenseitig Aufstellelemente 34 auf, die die Aufstellebene 26 definieren.

Die Abschnitte der Bodenwand 15 im Bereich von in Längsrichtung einander gegenüberliegenden Vertiefungen 32 sind durch Bodenabschnitte 36 miteinander verbunden, die längs der Längsseitenwände 17 und 18 verlaufen. Die Bodenabschnitte 36 erstrecken sich in Längsrichtung über ungefähr 60 % der Länge und in Querrichtung über ungefähr 25 % des Bodens 14. Die Bodenabschnitte 36 sind planar ausgestaltet und bilden Tragelemente 38, die eine parallel zur Aufstellebene 26 ausgerichtete Tragebene 40 definieren. Über die Tragebene 40 ragen keine Abschnitte der Bodenwand 14 hinaus. Auf den Tragelementen 38 kann eine Aufnahme für chirurgische Instrumente, insbesondere ein chirurgischer Siebkorb, zuverlässig stehend positioniert werden.

In Querrichtung sind die Bodenabschnitte im Bereich der Vertiefungen 32 längs der Querseitenwand 19 mittels eines Bodenabschnittes 44 miteinander verbunden. Längs der Querseitenwand 20 sind die Bodenabschnitte im Bereich der Vertiefungen 32 mittels eines Bodenabschnittes 45 miteinander verbunden.

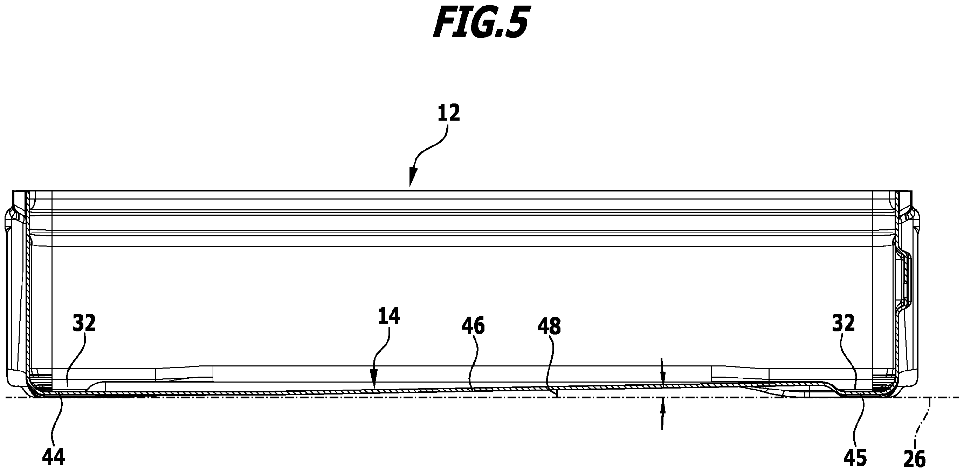

Die Bodenabschnitte 44 und 45 definieren eine gemeinsame Ebene, die parallel zur Aufstellebene 26 ausgerichtet ist und von dieser weniger weit beabstandet ist als von der Tragebene 40 (Figur 5). Der Bodenabschnitt 44 erstreckt sich in Längsrichtung über einen Bereich von ungefähr einem Viertel der Länge des Bodens 14, der Bodenabschnitt 45 über ungefähr 10% der Länge des Bodens 14. Im Abstand zur Querseitenwand 19 ist der Bodenabschnitt 44 mit einer Fluidableitfläche 46 des Bodens 14 verbunden, wobei die Verbindung in Querrichtung des Bodens 14 erfolgt.

Die Fluidableitfläche 46 erstreckt sich in Längsrichtung vom Bodenabschnitt 44 bis zum Bodenabschnitt 45 und in Querrichtung zwischen den Bodenabschnitten 36. Dabei überdeckt die Fluidableitfläche 46 die Mitte des Bodens 14, durch die die Symmetrieebene 28 und die Asymmetrieebene 30 verlaufen. Die Fluidableitfläche 46 ist planar ausgestaltet und in Draufsicht auf die Sterilisierbehälterwanne 12 ungefähr trapezförmig ausgebildet mit einer der Querseitenwand 20 zugewandten Basis. An der der Basis gegenüberliegenden Seite ist die Fluidableitfläche 46 mit dem Bodenabschnitt 44 verbunden. Insgesamt erstreckt sich die Fluidableitfläche 46 über ungefähr ein Drittel der Fläche des Bodens 14. Dabei überdeckt sie in Längsrichtung ungefähr 60 % bis ungefähr 70 % der Bodenfläche und in Querrichtung ungefähr 50 % der Bodenfläche.

Die Fluidableitfläche 46, insbesondere die von ihr definierte Ebene, ist relativ zur Aufstellebene 26 um einen Neigungswinkel 48 geneigt. Die Fluidableitfläche 46 schneidet die Aufstellebene nicht, da sie sich in Richtung auf die Aufstellebene nur bis zum Bodenabschnitt 44 erstreckt. Der Neigungswinkel 48 beträgt vorliegend weniger als 2°, beispielsweise ungefähr 1,5°. Die Fluidableitfläche 46 ist in Richtung auf die Querseitenwand 19 geneigt, so dass sie an ihrem der Querseitenwand 19 zugewandten Ende einen geringeren Abstand aufweist von der Aufstellebene 26 als an ihrem der Querseitenwand 19 gegenüberliegenden Ende. Letzteres Ende geht aus von der Tragebene 40, und das der Seitenwand 19 zugewandte Ende der Fluidableitfläche 46 ist in der vom Bodenabschnitt 44 definierten Ebene angeordnet.

Die Neigung der Fluidableitfläche 46 hat zur Folge, dass Fluid, insbesondere während des Sterilisiervorganges gebildetes Kondensat, von der Fluidableitfläche 46 in Richtung auf die Querseitenwand 19 abgeleitet wird. Da der Bodenabschnitt 44 und die Bodenwand 15 im Bereich der Vertiefungen 32 an der Querseitenwand 19 einen geringeren Abstand aufweisen von der Aufstellebene 26 als die Fluidableitfläche 46 (abgesehen von deren Verbindung mit dem Bodenabschnitt 44), wird Fluid dem Bodenabschnitt 44 und den Vertiefungen 32 an der Querseitenwand 19 zugeleitet. Dadurch bildet der Boden 14 im Bereich des Bodenabschnitts 44 und der Vertiefungen 32 an der Querseitenwand 19 einen Fluidsammelbereich 50 aus. Von der Fluidableitfläche 46 abgeleitetes Fluid sammelt sich im Fluidsammelbereich 50, wobei Fluid zunächst bis in die Vertiefungen 32 an der Querseitenwand 19 abgeleitet wird. Bei steigendem Fluidpegel kann sich Fluid auch auf dem Bodenabschnitt 44 sammeln, der bezüglich der Bodenwand 15 im Bereich der Vertiefungen 32 etwas erhöht liegt.

Bei vorstehend erwähnter Ausgestaltung verläuft der Fluidsammelbereich 50 entlang der Querseitenwand 19, die den Fluidsammelbereich 50 im Übergang zur Bodenwand 15 abschnittsweise begrenzt. Der Fluidsammelbereich 50 ist damit der Querseitenwand 19 benachbart. Auch von den Bodenabschnitten 36 kann Fluid in Längsrichtung über Abschrägungen der Bodenwand 15 in die Vertiefungen 32 an der Querseitenwand 19 geleitet werden.

Längs der Querseitenwand 20 weist der Boden 14 einen weiteren Fluidsammelbereich 52 auf. Der Fluidsammelbereich 52 wird gebildet durch die Vertiefungen 32 an der Querseitenwand 20 sowie den in Querrichtung dazwischen liegenden und vom Bodenabschnitt 45 untenseitig begrenzten Bereich. Von den Bodenabschnitten 36 kann Fluid auch in die Vertiefungen 32 an der Querseitenwand 20 abgeleitet werden. Insgesamt ist jedoch die Menge des sich im Fluidsammelbereich 52 sammelnden Fluids wesentlich geringer als die Menge des sich im Fluidsammelbereich 50 sammelnden Fluids. Dies ist auf die asymmetrische Ausgestaltung des Bodens 14 relativ zur Asymmetrieebene 30 und die geneigte Fluidableitfläche 46 zurückzuführen, mit der die überwiegende Anteil an Fluid in Richtung auf die Querseitenwand 19 in den Fluidsammelbereich 50 abgeleitet wird.

Um einen Medienaustausch von Medien wie Gas und/oder Fluid aus dem Behälterinnenraum 24 in die Umgebung und umgekehrt auch bei geschlossenem Sterilisierbehälter 10 zu ermöglichen, sind in der Außenwand 16 drei Durchgangsöffnungen vorgesehen. Die Deckenwand des Sterilisierbehälterdeckels 22 sowie der Boden 14 sind demgegenüber frei von Öffnungen.

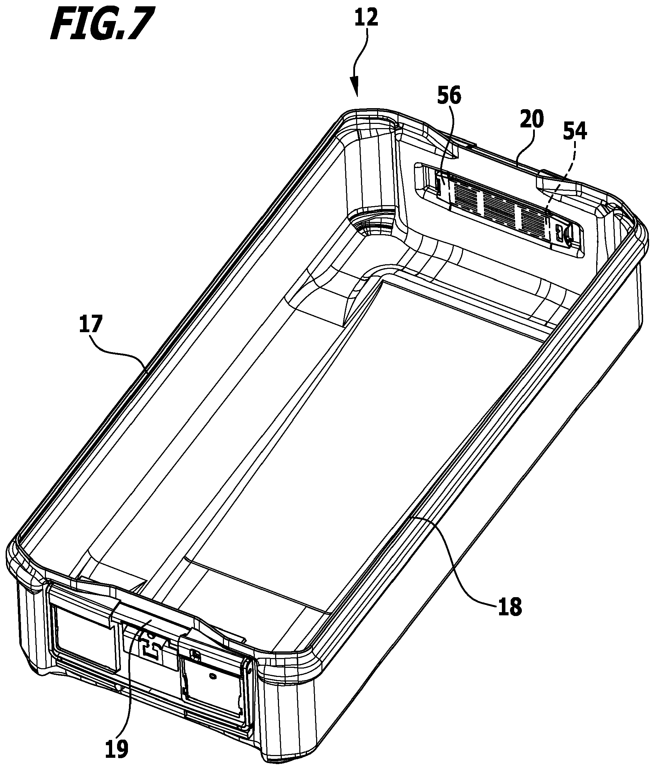

Eine erste Durchgangsöffnung ist, dies ist in der Zeichnung nicht dargestellt, in der Querseitenwand 20 gebildet. Die Durchgangsöffnung wird von einem Filter 54 überdeckt (Figur 7). Der Filter 54 wird mittels eines Filterhalteelementes 56 in Gestalt eines Haltebleches die Durchgangsöffnung überdeckend an der Querseitenwand 20 gehalten. Durch den Filter 54 hindurch erfolgt ein Medienaustausch zwischen der Umgebung und dem Behälterinnenraum 24, sofern eine erste maximale Druckdifferenz zwischen der Umgebung und dem Behälterinnenraum nicht überschritten bzw. eine zweite maximale Druckdifferenz zwischen dem Behälterinnenraum 24 und der Umgebung nicht überschritten wird.

Um in den letztgenannten Fällen einen Medienaustausch zu ermöglichen und eine Beschädigung des Filters 54 zu vermeiden, umfasst der Sterilisierbehälter 10 eine Ventileinrichtung 58. Die Ventileinrichtung 58 dient zum Freigeben und Verschließen von zwei Durchgangsöffnungen 60 und 62, die in der Querseitenwand 19 seitlich nebeneinander und im Abstand zum Boden 14 gebildet sind. Die Durchgangsöffnungen 60 sind vorliegend kreisförmig. Zum Verschließen und Freigeben der Durchgangsöffnungen 60 und 62 umfasst die Ventileinrichtung 58 ein Einlassventil 64 bzw. ein Auslassventil 66.

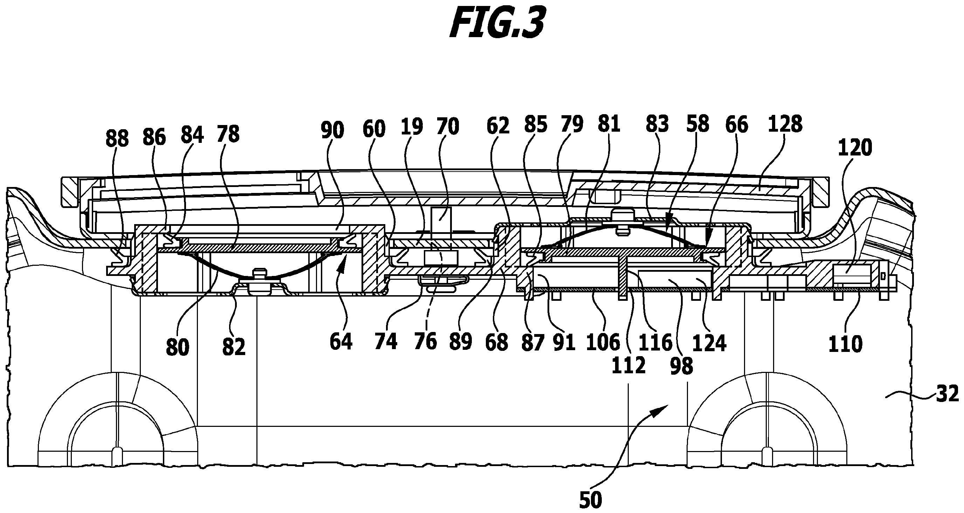

Den Ventilen 64 und 66 ist ein vorliegend einstückiges Ventilhalteteil 68 zugeordnet, an dem die Ventile 64 und 66 gehalten sind. Das Ventilhalteteil 68 ist im Wesentlichen plattenförmig ausgestaltet und mit der Querseitenwand 19 lösbar verbunden. Zu diesem Zweck sind Verbindungselemente in Gestalt von Nieten vorgesehen (ein Niet 70 ist in Figur 3 gezeigt). Der Niet 70 durchgreift eine Durchbrechung 72 im Ventilhalteteil 68, relativ zu dem er mittels eines Klemmelementes 74 kraft- und formschlüssig fixiert ist. Das Klemmelement 74 ist als Klemmschiene ausgestaltet, mittels derer beide Niete mit dem Ventilhalteteil 68 fixiert werden können. Mit dem freien Ende durchgreift der Niet 70 eine Durchbrechung 76 der Querseitenwand 19. Mit dem Rand der Durchbrechung 76 steht der Niet 70 in kraft- und formschlüssigem Eingriff, so dass er dadurch an der Querseitenwand 19 fixiert ist.

Zum Lösen des Ventilhalteteils 68 von der Querseitenwand 19 kann der kraftschlüssige Eingriff der Niete mit dem Klemmelement 74 aufgehoben werden und das Ventilhalteteil 68 mit den Ventilen 64 und 66 von der Sterilisierbehälterwanne 12 gelöst werden. Umgekehrt kann das Ventilhalteteil 68 mit der Sterilisierbehälterwanne 12 verbunden und dabei mit dem Klemmelement 74 an dieser fixiert werden.

Das Einlassventil 64 und das Auslassventil 66 sind druckbetätigte Ventile mit scheibenförmigen Ventilkörpern 78 bzw. 79, die sich mittels elastischer Rückstellelemente 80 bzw. 81 in Gestalt von Bügelfedern an mediendurchlässigen Ventilabdeckungen 82 bzw. 83 abstützen, die mit dem Ventilhalteteil 68 verbunden sind. Mittels Dichtelementen 84 bzw. 85, vorliegend in Gestalt von Lippendichtungen, können die Ventilkörper 78 bzw. 79 an Ventilsitzen 86 bzw. 87 des Einlassventils 64 bzw. des Auslassventils 66 dichtend anliegen. Die Ventilsitze 86 und 87 werden durch das Ventilhalteteil 68 ausgebildet. Über Dichtelemente 88 bzw. 89, vorliegend ebenfalls in Gestalt von Lippendichtungen, die die Ränder der Durchgangsöffnungen 60 und 62 umlaufen, ist das Ventilhalteteil dichtend in die Durchgangsöffnungen 60 und 62 eingesetzt. Dadurch verringert das Ventilhalteteil 68 die Querschnittsfläche der Durchgangsöffnungen 60 und 62. Soweit vorliegend von Freigeben bzw. Verschließen der Durchgangsöffnungen 60 und 62 durch das Einlassventil 62 bzw. das Auslassventil 66 die Rede ist, bezieht sich dies auf Durchgangsöffnungen 90 bzw. 91 im Ventilhalteteil 68, deren Ränder in die Durchgangsöffnungen 60 und 62 eingesetzt sind, so dass die Querschnitte der Durchgangsöffnungen 90 und 91 geringer sind als diejenigen der Durchgangsöffnungen 60 bzw. 62.

Der erfindungsgemäße Sterilisierbehälter 10 umfasst eine Fluidhebeeinrichtung 92, um Fluid aus dem Fluidsammelbereich 50 anzuheben und dem Auslassventil 66 zuzuführen. Fluid, insbesondere während des Sterilisiervorgangs gebildetes Kondensat, kann im Fluidsammelbereich 50, insbesondere in der Vertiefung 32 im Bereich der Querseitenwand 19 und der Längsseitenwand 17 gesammelt werden. Die Fluidhebeeinrichtung 92 stellt eine Fluidverbindung her zwischen dem Fluidsammelbereich 50 und dem Auslassventil 66, um Fluid aus dem Behälterinnenraum 24 zu entfernen.

Die Fluidhebeeinrichtung 92 umfasst zu diesem Zweck einen Fluidkanal 94 mit einer Kanaleintrittsöffnung 96 und einer Kanalaustrittsöffnung 98. Der Fluidkanal 94 weist einen die Kanaleintrittsöffnung 96 bildenden ersten Kanalabschnitt 100 sowie einen die Kanalaustrittsöffnung 98 bildenden zweiten Kanalabschnitt 102 auf.

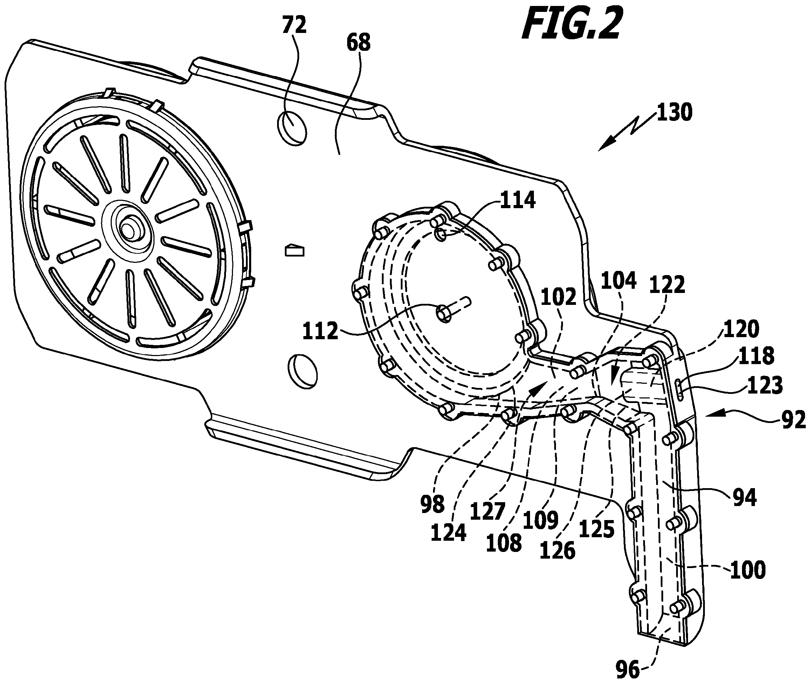

Der erste Kanalabschnitt 100 greift in die Vertiefung 32 am Eckbereich der Querseitenwand 19 mit der Längsseitenwand 17 und damit in den Fluidsammelbereich 50 ein, wobei er die Bodenwand 15 jedoch nicht kontaktiert. Der Abstand des Kanalabschnitts 100 zur Bodenwand beträgt beispielsweise ungefähr 1 bis 5 mm. Relativ zur Aufstellebene 26 ist der erste Kanalabschnitt 100 senkrecht ausgerichtet und erstreckt sich von dieser wegweisend nach oben ungefähr bis zur halben Höhe der Querseitenwand 19. Die Kanaleintrittsöffnung 96 ist auf die Bodenwand 15 gerichtet, worunter verstanden werden kann, dass die der Durchtrittsrichtung von Fluid durch die Kanaleintrittsöffnung 96 entgegengesetzte Richtung auf die Bodenwand 15 weist (Figur 1). Die Eintrittsöffnung 96 weist eine Neigung bezüglich der Bodenwand 15 auf infolge einer Abschrägung des ersten Kanalabschnitts 100 an dem der Bodenwand 15 zugewandten Ende (Figur 2).

Der zweite Kanalabschnitt 102 und der erste Kanalabschnitt 100 sind in einem Winkel relativ zueinander ausgerichtet, der vorliegend kleiner ist als 90°. Eine vom zweiten Kanalabschnitt 102 definierte Richtung ist relativ zur Aufstellebene 26 geneigt, so dass der zweite Kanalabschnitt 102 in Richtung der Aufstellebene 26 abfällt. Die Kanalaustrittsöffnung 98 ist an einer dem Boden 14 zugewandten Seite des Auslassventils 66 angeordnet. Die Kanalaustrittsöffnung 98 ist auf die Aufstromseite des Auslassventils 66 gerichtet, wobei die Durchtrittsrichtung von Fluid durch die Kanalaustrittsöffnung 98 im Wesentlichen parallel zu einer vom scheibenförmigen Ventilkörper 79 definierten Ebene ist.

Der zweite Kanalabschnitt 102 weist eine Kanalverengung 104 auf, im Bereich derer die Querschnittsfläche des Fluidkanals 94 verkleinert ist. In Strömungsrichtung des Fluids durch den Fluidkanal 94 nachgelagert erweitert sich der Querschnitt des Fluidkanals 94 nach der Kanalverengung 104 bis zur Kanalaustrittsöffnung 98 wieder.

Der Fluidkanal 94 wird gebildet durch Kanalwände, die ausgebildet werden vom Ventilhalteteil 68 sowie einer mit diesem verbundenen Abdeckung 106. Im Ventilhalteteil 68 ist zu diesem Zweck eine Nut 108 gebildet, die eine erste Kanalwand 109 des Fluidkanals 94 bildet. Die der Kanalwand 109 gegenüberliegende Kanalwand 110 wird gebildet durch die Abdeckung 106. Die Kanalwände 109 und 110 werden durch seitliche Nutwände der Nut 108 vom Ventilhalteteil 68 gebildet.

Die Abdeckung 106 überdeckt die Nut 108 und die Durchgangsöffnung 91 im Ventilhalteteil 68 und damit das Auslassventil 66 behälterinnenseitig. Der Fluidkanal 94 verläuft aufgrund der vorstehend erwähnten Ausgestaltung zwischen dem Ventilhalteteil 68 und der vorliegend plattenförmigen Abdeckung 106 im Wesentlichen in einer bzw. parallel zu einer vom Ventilhalteteil 68 definierten Ebene.

Aufgrund der starren Ausgestaltung des Ventilhalteteils 68 und der Abdeckung 106 ist der Fluidkanal 94 formstabil. Über das Ventilhalteteil 68 ist der Fluidkanal 94 mit der Querseitenwand 19 lösbar verbunden.

Die Abdeckung 106 weist in ihrem die Durchgangsöffnung 91 überdeckenden Abschnitt zwei Durchbrechungen 112 und 114 auf. In die zentrale Durchbrechung 112 greift ein vorliegend zapfenförmiger Vorsprung 116 am Ventilkörper 79 ein, wenn das Auslassventil 66 geschlossen ist. Der Vorsprung 116 und die Durchbrechung 112 dienen damit als zusammenwirkende Ausrichtelemente zur Ausrichtung des Ventilkörpers 79 relativ zur Abdeckung 106. Die Durchbrechung 114 ist oberhalb der Durchbrechung 112 angeordnet und liegt dem Außenrand des Ventilkörpers 79 behälterinnenseitig gegenüber.

Im Übergangsbereich vom ersten Kanalabschnitt 100 zum zweiten Kanalabschnitt 102 weist der Fluidkanal 94 eine Eintrittsöffnung 118 auf, in Verlängerung der vom zweiten Kanalabschnitt 102 definierten Richtung. Ein hülsenförmiger Einsatz 120, dessen Durchgang mit der Eintrittsöffnung 118 fluchtet, ist im Fluidkanal 94 angeordnet, im Abzweigungsbereich des zweiten Kanalabschnitts 102 vom ersten Kanalabschnitt 100. Bezogen auf die Strömungsrichtung von Fluid durch den Fluidkanal 94 sind der Einsatz 120 und damit die Eintrittsöffnung 118 der Kanalverengung 104 vorgelagert.

Die Fluidhebeeinrichtung 92 umfasst einen in den Fluidkanal 94 integrierten Injektor 122 mit einer Injektoreintrittsöffnung 123, einer Injektoraustrittsöffnung 124 und einer Saugöffnung 125. Die Injektoreintrittsöffnung 123 wird ausgebildet durch die Eintrittsöffnung 118 des Fluidkanals 94, und die Injektoraustrittsöffnung 124 wird gebildet durch die Kanalaustrittsöffnung 98. Die Saugöffnung 125 wird gebildet durch den Fluidkanal 94 in dessen den Einsatz 120 umgebenden Bereich. Der Einsatz 120 hat die Funktion einer Düse 126 des Injektors 122. Der Injektor umfasst ferner einen Diffusor 127, der in den Kanal integriert ist und abschnittsweise vom zweiten Kanalabschnitt 102 gebildet wird. Der Diffusor 127 erstreckt sich von der Kanalverengung 104 bis zur Kanalaustrittsöffnung 98.

Nachfolgend wird auf Zweck und Funktion der Fluidhebeeinrichtung 92 in Kombination mit dem Auslassventil 66 eingegangen. Die Fluidhebeeinrichtung 92 ist vorgesehen, um eine Flüssigkeit, insbesondere Kondensat, am Ende des Sterilisiervorgangs aus dem Behälterinnenraum 24 zu entfernen. Dabei wird der Umgebungsdruck um den Sterilisierbehälter 10 so weit erniedrigt, bis er den Innendruck des Sterilisierbehälters 10 deutlich unterschreitet. Infolge der Druckdifferenz öffnet das druckgesteuerte Auslassventil 66, so dass ein Druckausgleich mit dem Umgebungsdruck erfolgen kann. Gas und Fluid können aus dem Sterilisierbehälter 10 austreten.

Zum Öffnen des Auslassventils 66 erweist es sich als vorteilhaft, dass durch die Durchbrechungen 112 und 114 der Abdeckung 106 und durch die Eintrittsöffnung 118 im Fluidkanal 94 Strömungsverbindungen zwischen dem Auslassventil 66 und dem Behälterinnenraum 24 gebildet sind, bei hinreichend hohem Kondensatpegel in der Vertiefung 32 unter Umgehung des ersten Kanalabschnitts 100. Aufgrund des erhöhten Behälterinnendrucks können sich durch die Eintrittsöffnung 118 und den zweiten Kanalabschnitt 102 sowie durch die Durchbrechungen 112 und 114 hindurch unter Öffnen des Auslassventils 66 Nebenstrompfade am ersten Kanalabschnitt 100 vorbei ausbilden.

Je nach Höhe des Kondensatpegels im Fluidsammelbereich 50 ist es jedoch auch möglich, dass sich eine Gasströmung durch den ersten Kanalabschnitt 100 ausbildet, wenn der Kondensatpegel so niedrig ist, dass der erste Kanalabschnitt 100 mit der Kanaleintrittsöffnung 96 nicht vollständig in Fluid eintaucht.

Öffnet das Auslassventil 66, entsteht durch den Injektor 122 eine Saugströmung, indem Gas durch die Eintrittsöffnung 118 und die Düse 126 sowie den zweiten Kanalabschnitt 102 zum Auslassventil 66 strömt. Infolge der Kanalverengung 104 erfolgt eine Druckminderung an der Saugöffnung 125, so dass sich eine Druckdifferenz im Fluidkanal 94 zwischen der Saugöffnung 125 und dem Druck an der Kanaleintrittsöffnung 96 ausbildet. Dies führt dazu, dass Fluid aus dem Fluidsammelbereich in den Fluidkanal 94 eingesaugt wird. Weiter wird Fluid durch den Fluidkanal 94 angehoben und dem Auslassventil 66 zugeführt, unter andauernder Saugströmung durch den Injektor 122.

Als vorteilhaft erweist es sich dabei, dass der zweite Kanalabschnitt 102 in Richtung auf die Aufstellebene 26 geneigt ist. Dies führt dazu, dass sich die Strömung angehobenen Fluids beruhigt, so dass es nach Verlassen des Fluidkanals 94 nicht sprühstoßartig aus dem Sterilisierbehälter 10 austritt. Zu diesem Zweck ist auch der Diffusor 127 zwischen der Kanalverengung 104 und der Kanalaustrittsöffnung 98 vorgesehen, mit dem eine Drucksteigerung bei gleichzeitiger Beruhigung der Strömung des Fluids sichergestellt wird. Zum Schutz von Versprühen von austretendem Fluid ist ferner ein im Wesentlichen plattenförmiges Abdeckelement 128 an der Außenseite der Querseitenwand 19 gehalten. Das Abdeckelement 128 dient ferner zur Aufnahme und Lagerung eines Behältergriffs des Sterilisierbehälters 10.

Das Anheben von Fluid aus dem Fluidsammelbereich 50 durch den Fluidkanal 94 hindurch unter der Wirkung der Saugströmung durch den Injektor 122 ist auch dann möglich und wirksam, wenn der Fluidpegel so weit abgefallen ist, dass der erste Kanalabschnitt 100 nicht vollständig mit der Kanaleintrittsöffnung 96 in das Fluid eintaucht. Auch bei einer gemischten Kondensat- und Gasströmung durch den ersten Kanalabschnitt 100 hindurch zeigt sich, dass unter der Wirkung der Saugströmung durch den Injektor 122 weiterhin Fluid wirkungsvoll aus dem Fluidsammelbereich 50 angehoben und dem Auslassventil 66 zugeführt werden kann.

Weiter erweist es sich, insbesondere bei hohem, die Kanaleintrittsöffnung 96 übersteigenden Kondensatpegel als vorteilhaft, dass durch die Durchbrechungen 112 und 114 und die Eintrittsöffnung 118 Nebenstrompfade am ersten Kanalabschnitt 100 vorbei vorhanden sind. Dies führt zu einem Druckabbau im Behälterinnenraum 24 über mehr als nur einen Strömungspfad, so dass Fluid nicht schwallartig durch den Fluidkanal 94 abgesaugt und aus dem Sterilisierbehälter 10 entfernt wird. Auch die mechanische Belastung des Sterilisierbehälters 10 kann dadurch verringert werden.

Nach Schließen des Auslassventils 66 im Sterilisierbehälter 10 möglicherweise noch vorhandenes Fluid kann während der Trocknungsphase nach dem eigentlichen Sterilisiervorgang durch die Restwärme speziell der Sterilisierbehälterwanne 12 verdampfen und durch den Filter 54 hindurch aus dem Behälterinnenraum 24 austreten. Hierzu erweist es sich als vorteilhaft, dass der Fluidsammelbereich 50 längs der Querseitenwand 19 verläuft, so dass auch die in der Querseitenwand 19 gespeicherte Restwärme zum Verdampfen von Fluid wirksam ist. Entsprechend verhält es sich mit dem Fluidsammelbereich 52, der längs der Querseitenwand 20 verläuft.

Das Vorsehen der Fluidhebeeinrichtung 92 erweist sich als vorteilhaft, um eine große Menge an Fluid wirksam anzuheben und aus dem Behälterinnenraum 24 zu entfernen, noch bevor die eigentliche Trocknungsphase des Sterilisiervorganges einsetzt. Die Trocknungsphase kann dadurch wesentlich verkürzt werden.

Das Vorsehen der Ableitfläche 46 und des Fluidsammelbereiches erlaubt es, einen großen Anteil des sich bildenden Fluids, insbesondere Kondensats, an der Querseitenwand 19 zu sammeln, so dass es an dieser von der Fluidhebeeinrichtung 92 angehoben und dem Auslassventil 66 zugeführt werden kann.

Weiter ist es besonders günstig, dass der Boden 14 frei von jeglichen Durchgangsöffnungen ist. Auf Ventile zum Verschließen des Bodens 14 kann dadurch verzichtet werden, und der Boden 14 bildet eine Sterilbarriere. Die Gefahr eines Eindringens von Keimen durch Durchgangsöffnungen im Boden, wie dies bei Sterilisierbehältern mit Fluidablassventilen im Boden der Fall ist, kann dadurch auch nach dem Ende des Sterilisiervorgangs vermieden werden. Auch eine Beschädigung oder Fehlfunktion eines Fluidablassventils kann verhindert werden.

Das Auslassventil 66 und die Fluidhebeeinrichtung 92 sind vorliegend Bestandteil einer bevorzugten Ausführungsform einer in Figur 2 perspektivisch dargestellten und mit dem Bezugszeichen 130 belegten chirurgischen Fluidextraktionsvorrichtung, die beim Sterilisierbehälter 10 zum Einsatz kommt. Die Fluidextraktionsvorrichtung 130 umfasst ferner das Ventilhalteteil 68 sowie das Einlassventil 64. Es kann alternativ vorgesehen sein, dass das Einlassventil 64 nicht im Ventilhalteteil 68 gehalten ist und dementsprechend kein Bestandteil der Fluidextraktionsvorrichtung 130 ist. Wie erwähnt, kann die Fluidextraktionsvorrichtung 130 lösbar mit der Querseitenwand 19 verbunden werden, was über eine Montageerleichterung hinaus auch deren Austausch bei Bedarf ermöglicht.

## Patentansprüche

1. Chirurgische Sterilisierbehälterwanne, auf die ein Sterilisierbehälterdeckel (22) lösbar aufsetzbar ist zum Ausbilden eines chirurgischen Sterilisierbehälters (10), in dem chirurgische Instrumente zum Sterilisieren aufnehmbar sind, wobei die Sterilisierbehälterwanne (12) einen Boden (14) sowie eine vom Boden (14) abstehende Behälterwand (16) umfasst, wobei die Sterilisierbehälterwanne (12) eine Aufstellebene (26) definiert und wobei der Boden (14) einen Fluidsammelbereich (50) zum Sammeln von Fluid umfasst oder ausbildet, **dadurch gekennzeichnet, dass** der Boden (14) eine relativ zur Aufstellebene (26) geneigte und mit dem Fluidsammelbereich (50) in Fluidverbindung stehende Fluidableitfläche (46) zum Ableiten von Fluid in Richtung der Behälterwand (16) umfasst, dass die Größe der Fluidableitfläche (46) mindestens 20 % der Bodenfläche beträgt, dass die Sterilisierbehälterwanne (12) einen rechteckigen Querschnitt aufweist mit vier die Behälterwand (16) bildenden Seitenwänden (17, 18, 19, 20), wobei die Fluidableitfläche (46) in Richtung einer der Seitenwände (17, 18, 19, 20) geneigt ist zum Ableiten von Fluid in Richtung der Seitenwand (17, 18, 19, 20), und dass der Fluidsammelbereich (50) derjenigen Seitenwand (17) benachbart ist, in Richtung derer die Fluidableitfläche (46) zum Ableiten von Fluid geneigt ist.

2. Sterilisierbehälterwanne nach Anspruch 1, **dadurch gekennzeichnet, dass** der Boden (14) am Fluidsammelbereich (50) frei von Durchgangsöffnungen ist.

3. Sterilisierbehälterwanne nach Anspruch 1 oder 2, **dadurch gekennzeichnet, dass** der Boden (14) frei von Durchgangsöffnungen ist.

4. Sterilisierbehälterwanne nach einem der voranstehenden Ansprüche, **dadurch gekennzeichnet, dass** die Größe der Fluidableitfläche (46) mindestens 30 % der Bodenfläche beträgt.

5. Sterilisierbehälterwanne nach einem der voranstehenden Ansprüche, **dadurch gekennzeichnet, dass** die Neigung der Fluidableitfläche (46) relativ zur Aufstellebene (26) kleiner ist als 10°, bevorzugt kleiner als 5°, noch bevorzugter kleiner als 2°.

6. Sterilisierbehälterwanne nach einem der voranstehenden Ansprüche, **dadurch gekennzeichnet, dass** der Fluidsammelbereich (50) durch eine Vertiefung des Bodens (14) gebildet ist.

7. Sterilisierbehälterwanne nach einem der voranstehenden Ansprüche, **dadurch gekennzeichnet, dass** die Sterilisierbehälterwanne (12) eine den Boden (14) bildende Bodenwand (15) umfasst und dass die Bodenwand (15) die Fluidableitfläche (46) und/oder den Fluidsammelbereich (50) ausbildet.

8. Sterilisierbehälterwanne nach einem der voranstehenden Ansprüche, **dadurch gekennzeichnet, dass** der Fluidsammelbereich (50) in direkter Fluidverbindung mit der Behälterwand (16) steht.

9. Sterilisierbehälterwanne nach einem der voranstehenden Ansprüche, **dadurch gekennzeichnet, dass** der Fluidsammelbereich (50) längs eines Abschnittes der Behälterwand (16) verläuft.

10. Sterilisierbehälterwanne nach einem der voranstehenden Ansprüche, **dadurch gekennzeichnet, dass** sich die Fluidableitfläche (46) über die Mitte des Bodens (14) hinweg erstreckt.

11. Sterilisierbehälterwanne nach einem der voranstehenden Ansprüche, **dadurch gekennzeichnet, dass** die Sterilisierbehälterwanne (12) eine senkrecht zur Aufstellebene (26) ausgerichtete Wannenmittenebene (30) definiert, bezüglich derer der Boden (14) asymmetrisch ausgebildet ist, vorzugsweise dass sich die Fluidableitfläche (46) über eine Mitte des Bodens (14) erstreckt und die Wannenmittenebene (30) schneidet.

12. Sterilisierbehälterwanne nach einem der voranstehenden Ansprüche, **dadurch gekennzeichnet, dass** die Sterilisierbehälterwanne (12) eine senkrecht zur Aufstellebene (26) ausgerichtete Wannenmittenebene (28) definiert, bezüglich derer der Boden (14) symmetrisch ausgebildet ist.

13. Sterilisierbehälterwanne nach einem der voranstehenden Ansprüche, **dadurch gekennzeichnet, dass** der Boden (14) Vertiefungen (32) umfasst oder ausbildet, im Bereich derer die Sterilisierbehälterwanne (12) außenseitig Aufstellelemente (34) aufweist, die die Aufstellebene (26) definieren.

14. Sterilisierbehälterwanne nach Anspruch 13, **dadurch gekennzeichnet, dass** die Vertiefungen (32), die einem Abschnitt der Behälterwand (16) benachbart sind, in Richtung auf den die Ableitfläche (46) geneigt ist, einen Teil des Fluidsammelbereiches (50) ausbilden.

15. Chirurgischer Sterilisierbehälter, umfassend eine chirurgische Sterilisierbehälterwanne nach einem der voranstehenden Ansprüche und einen auf diese lösbar aufsetzbaren Sterilisierbehälterdeckel.

## Claims

1. Surgical sterilizing container tub on which a sterilizing container lid (22) is adapted to be detachably fitted for formation of a surgical sterilizing container (10) for holding surgical instruments for sterilization, the sterilizing container tub (12) comprising a bottom (14) and a container wall (16) projecting from the bottom (14), the sterilizing container tub (12) defining a set-down plane (26), and the bottom (14) comprising or forming a fluid collection area (50) for collecting fluid, **characterized in that** the bottom (14) comprises a fluid drainage surface (46) which is inclined relative to the set-down plane (26) and is in fluid connection with the fluid collection area (50) for draining fluid in the direction of the container wall (16), **in that** the size of the fluid drainage surface (46) is at least 20% of the bottom surface, **in that** the sterilizing container tub (12) is of rectangular cross section with four side walls (17, 18, 19, 20) forming the container wall (16), the fluid drainage surface (46) is inclined in the direction of one of the side walls (17, 18, 19, 20) for draining fluid in the direction of the side wall (17, 18, 19, 20), and **in that** the fluid collection area (50) is adjacent to that side wall (17) in the direction of which the fluid drainage surface (46) is inclined for draining off fluid.

2. Sterilizing container tub in accordance with claim 1, **characterized in that** the bottom (14) is free of through-openings at the fluid collection area (50).

3. Sterilizing container tub in accordance with claim 1 or 2, **characterized in that** the bottom (14) is free of through-openings.

4. Sterilizing container tub in accordance with any one of the preceding claims, **characterized in that** the size of the fluid drainage surface (46) is at least 30 % of the bottom surface.

5. Sterilizing container tub in accordance with any one of the preceding claims, **characterized in that** the inclination of the fluid drainage surface (46) relative to the set-down plane (26) is less than 10°, preferably less than 5°, even more preferred less than 2°.

6. Sterilizing container tub in accordance with any one of the preceding claims, **characterized in that** the fluid collection area (50) is formed by a depression in the bottom (14).

7. Sterilizing container tub in accordance with any one of the preceding claims, **characterized in that** the sterilizing container tub (12) comprises a bottom wall (15) forming the bottom (14), and **in that** the bottom wall (15) forms the fluid drainage surface (46) and/or the fluid collection area (50).

8. Sterilizing container tub in accordance with any one of the preceding claims, **characterized in that** the fluid collection area (50) is in direct fluid connection with the container wall (16).

9. Sterilizing container tub in accordance with any one of the preceding claims, **characterized in that** the fluid collection area (50) extends along a section of the container wall (16).

10. Sterilizing container tub in accordance with any one of the preceding claims, **characterized in that** the fluid drainage surface (46) extends beyond the center of the bottom (14).

11. Sterilizing container tub in accordance with any one of the preceding claims, **characterized in that** the sterilizing container tub (12) defines a tub center plane (30) which is aligned perpendicularly to the set-down plane (26) and in relation to which the bottom (14) is asymmetrically configured, preferably that the fluid drainage surface (46) extends beyond a center of the bottom (14) and intersects the tub center plane (30).

12. Sterilizing container tub in accordance with any one of the preceding claims, **characterized in that** the sterilizing container tub (12) defines a tub center plane (28) which is aligned perpendicularly to the set-down plane (26) and in relation to which the bottom (14) is symmetrically configured.

13. Sterilizing container tub in accordance with any one of the preceding claims, **characterized in that** the bottom (14) comprises or forms depressions (32) in the area of which the sterilizing container tub (12) has set-down elements (34) on the outside, which define the set-down plane (26).

14. Sterilizing container tub in accordance with claim 13, **characterized in that** the depressions (32), which are adjacent to a section of the container wall (16) in the direction of which the drainage surface (46) is inclined, form part of the fluid collection area (50).

15. Surgical sterilizing container, comprising a surgical sterilizing container tub in accordance with any one of the preceding claims and a sterilizing container lid adapted to be detachably fitted thereon.

## Revendications

1. Bac de contenant de stérilisation chirurgical, sur lequel peut être appliqué de manière amovible, un couvercle de contenant de stérilisation (22), en vue de former un contenant de stérilisation chirurgical (10) dans lequel peuvent être reçus des instruments chirurgicaux à stériliser, le bac de contenant de stérilisation (12) comprenant un fond (14) ainsi qu'une paroi de contenant (16) issue du fond (14), le bac de contenant de stérilisation (12) définissant également un plan d'appui (26), et le fond (14) comportant ou formant une zone de collecte de fluide (50) pour collecter du fluide,
**caractérisé en ce que** le fond (14) comprend une surface d'évacuation de fluide (46), qui est inclinée par rapport au plan d'appui (26) et est en liaison d'écoulement de fluide avec la zone de collecte de fluide (50), et qui est destinée à évacuer du fluide en direction de la paroi de contenant (16), **en ce que** la grandeur de la surface d'évacuation de fluide (46) est d'au moins 20% celle de la surface de fond, **en ce que** le bac de contenant de stérilisation (12) présente une section rectangulaire avec quatre parois latérales (17, 18, 19, 20) formant la paroi de contenant (16), la surface d'évacuation de fluide (46) étant inclinée en direction de l'une des parois latérales (17, 18, 19, 20) pour évacuer du fluide en direction de la paroi latérale (17, 18, 19, 20), et **en ce que** la zone de collecte de fluide (50) est voisine de la paroi latérale (17) en direction de laquelle est inclinée la surface d'évacuation de fluide (46) pour évacuer du fluide.

2. Bac de contenant de stérilisation selon la revendication 1, **caractérisé en ce que** le fond (14) est exempt d'ouvertures de passage dans la zone de collecte de fluide (50).

3. Bac de contenant de stérilisation selon la revendication 1 ou la revendication 2, **caractérisé en ce que** le fond (14) est exempt d'ouvertures de passage.

4. Bac de contenant de stérilisation selon l'une des revendications précédentes, **caractérisé en ce que** la grandeur de la surface d'évacuation de fluide (46) est d'une grandeur d'au moins 30% de celle de la surface de fond.

5. Bac de contenant de stérilisation selon l'une des revendications précédentes, **caractérisé en ce que** l'inclinaison de la surface d'évacuation de fluide (46) par rapport au plan d'appui (26) est inférieure à 10°, de préférence inférieure à 5°, et de manière plus préférée encore, inférieure à 2°.

6. Bac de contenant de stérilisation selon l'une des revendications précédentes, **caractérisé en ce que** la zone de collecte de fluide (50) est formée par un creux du fond (14).

7. Bac de contenant de stérilisation selon l'une des revendications précédentes, **caractérisé en ce que** le bac de contenant de stérilisation (12) comprend une paroi de fond (15) formant le fond (14), et **en ce que** la paroi de fond (15) forme la surface d'évacuation de fluide (46) et/ou la zone de collecte de fluide (50).

8. Bac de contenant de stérilisation selon l'une des revendications précédentes, **caractérisé en ce que** la zone de collecte de fluide (50) est en liaison d'écoulement de fluide directe avec la paroi de contenant (16).

9. Bac de contenant de stérilisation selon l'une des revendications précédentes, **caractérisé en ce que** la zone de collecte de fluide (50) s'étend le long d'un secteur de la paroi de contenant (16).

10. Bac de contenant de stérilisation selon l'une des revendications précédentes, **caractérisé en ce que** la surface d'évacuation de fluide (46) s'étend par-delà le milieu du fond (14).

11. Bac de contenant de stérilisation selon l'une des revendications précédentes, **caractérisé en ce que** le bac de contenant de stérilisation (12) définit un plan médian de bac (30) orienté perpendiculairement au plan d'appui (26) et par rapport auquel le fond (14) est de configuration asymétrique, de préférence **en ce que** la surface d'évacuation de fluide (46) s'étend par-delà un milieu du fond (14) et coupe ledit plan médian de bac (30) .

12. Bac de contenant de stérilisation selon l'une des revendications précédentes, **caractérisé en ce que** le bac de contenant de stérilisation (12) définit un plan médian de bac (28) orienté perpendiculairement au plan d'appui (26) et par rapport auquel le fond (14) est de configuration symétrique.

13. Bac de contenant de stérilisation selon l'une des revendications précédentes, **caractérisé en ce que** le fond (14) comporte ou forme des creux (32) dans la zone desquels le bac de contenant de stérilisation (12) présente sur le côté extérieur, des éléments d'appui (34), qui définissent le plan d'appui (26).

14. Bac de contenant de stérilisation selon la revendication 13, **caractérisé en ce que** les creux (32), qui sont voisins d'un secteur de la paroi de contenant (16) en direction duquel est inclinée la surface d'évacuation de fluide (46), forment une partie de la zone de collecte de fluide (50).

15. Contenant de stérilisation chirurgical, comprenant un bac de contenant de stérilisation chirurgical selon l'une des revendications précédentes, et un couvercle de contenant de stérilisation pouvant y être appliqué de manière amovible.
